# EUROPÄISCHE PATENTANMELDUNG

(11) **EP 4 623 863 A2**
(43) Veröffentlichungstag der Anmeldung: **01.10.2025**
(21) Anmeldenummer: 25162501.8
(22) Anmeldetag: 10.03.2025
(51) Int. Cl.: A61C 19/00, A61B 90/70, A61L 2/18, A61L 2/22

(54) **REINIGUNGS- UND/ODER DESINFEKTIONSGERÄT**

(30) Priorität: 27.03.2024 LU 103271
(71) Anmelder: Miele & Cie. KG, 33332 Gütersloh (DE)
(72) Erfinder: Werner, Alexander, 33619 Bielefeld (DE); Goldberg, Gregor, 48231 Warendorf (DE); Crilley, Ivana, 33335 Gütersloh (DE)

(57) **Zusammenfassung**

Die Erfindung betrifft ein Reinigungs- und/oder Desinfektionsgerät zur Aufbereitung eines medizinischen, insbesondere zahnmedizinischen Hohlkörperinstruments, mit einem einen Behandlungsraum (2) bereitstellenden Gerätebehälter (3), der der Aufnahme des aufzubereitenden Hohlkörperinstruments (45) dient, mit einer innerhalb des Gerätebehälters (3) angeordneten Sprüheinrichtung (4) zur Außenbeaufschlagung des Hohlkörperinstruments (45) mit einem Reinigungs- und/oder Desinfektionsmittel, und mit einer innerhalb des Gerätebehälters (3) angeordneten Beschickungseinrichtung (32) zur Innenbeaufschlagung eines vom Hohlkörperinstrument (45) bereitgestellten Hohlraums, insbesondere eines englumigen Kanals oder dgl., mit dem Reinigungs- und/oder Desinfektionsmittel, gekennzeichnet durch eine mit der Beschickungseinrichtung (32) in strömungstechnischer Wirkverbindung stehende Medienschnittstelle (29), die medieneingangsseitig an eine Zuführleitung (25, 44) für das Reinigungs- und/oder Desinfektionsmittel, an eine Zuführleitung (19, 44) für Druckluft und an eine Zuführleitung (22) für ein Pflegemittel strömungstechnisch angeschlossen ist.

## Beschreibung

Die Erfindung betrifft ein Reinigungs- und/oder Desinfektionsgerät zur Aufbereitung eines medizinischen, insbesondere zahnmedizinischen Hohlkörperinstruments, mit einem einen Behandlungsraum bereitstellenden Gerätebehälter, der der Aufnahme des aufzubereitenden Hohlkörperinstruments dient, mit einer innerhalb des Gerätebehälters angeordneten Sprüheinrichtung zur Außenbeaufschlagung des Hohlkörperinstruments mit einem Reinigungs- und/oder Desinfektionsmittel, und mit einer innerhalb des Gerätebehälters angeordneten Beschickungseinrichtung zur Innenbeaufschlagung eines vom Hohlkörperinstrument bereitgestellten Hohlraums, insbesondere eines englumigen Kanals oder dgl., mit dem Reinigungs- und/oder Desinfektionsmittel.

Reinigungs- und/oder Desinfektionsgeräte der vorgenannten Art sind aus dem Stand der Technik an sich gut bekannt, weshalb es eines gesonderten druckschriftlichen Nachweises an dieser Stelle nicht bedarf. Es sei deshalb auch nur beispielhaft auf die DE 10 2020 134 621 A1 verwiesen, die ein gattungsgemäßes Reinigungs- und/oder Desinfektionsgerät offenbart.

Ein gattungsgemäßes Reinigungs- und/oder Desinfektionsgerät verfügt über einen Gerätebehälter, der seinerseits einen Behandlungsraum bereitstellt. Dieser dient im bestimmungsgemäßen Verwendungsfall der Aufnahme von zu reinigenden und/oder zu desinfizierenden Spülgütern im Allgemeinen sowie der Aufnahme von aufzubereitenden Hohlkörperinstrumenten im Speziellen. Bei "Hohlkörperinstrumenten" handelt es sich um medizinische Instrumente, insbesondere zahnmedizinischen Instrumente, wobei der vom Hohlkörperinstrument bereitgestellte Hohlraum englumig ausgebildet ist, beispielsweise in der Ausgestaltung eines Kanals, einer Leitung und/oder dgl. "Hohlkörperinstrumente" in diesem Sinne sind beispielsweise dentaltechnische Übertragungsinstrumente, insbesondere Winkelstücke, Handstücke, Turbinen und/oder dgl.

Innerhalb des Gerätebehälters ist eine Sprüheinrichtung angeordnet, die der Außenbeaufschlagung eines Hohlkörperinstruments mit einem Reinigungs- und/oder Desinfektionsmittel dient. Die Sprüheinrichtung verfügt zu diesem Zweck typischerweise über sich im bestimmungsgemäßen Verwendungsfall verdrehende Sprüharme, wobei zwei oder drei solcher Sprüharme vorgesehen sein können. Im bestimmungsgemäßen Verwendungsfall findet mittels der Sprüheinrichtung eine Außenreinigung und/oder Desinfektion der vom Behandlungsraum aufgenommenen Spülgüter, insbesondere Hohlkörperinstrumente, statt.

Ein gattungsgemäßes Reinigungs- und/oder Desinfektionsgerät verfügt des Weiteren über eine innerhalb des Gerätebehälters angeordnete Beschickungseinrichtung. Diese Beschickungseinrichtung dient der Innenbeaufschlagung eines von einem Hohlkörperinstrument bereitgestellten Hohlraums, insbesondere eines englumigen Kanals und/oder dgl., und zwar mit dem auch zur Außenreinigung genutzten Reinigungs- und/oder Desinfektionsmittels.

Im bestimmungsgemäßen Verwendungsfall erlaubt ein gattungsgemäßes Reinigungs- und/oder Desinfektionsgerät sowohl eine Außenreinigung als auch eine Innenreinigung eines Hohlkörperinstruments, einschließlich einer Außendesinfektion und einer Innendesinfektion.

Als Reinigungs- und/oder Desinfektionsmittel kann insbesondere Wasser, vorzugsweise Frischwasser Verwendung finden. In Ergänzung hierzu kann eine Prozesschemikalie Verwendung finden, insbesondere als Zugabe zum eingesetzten Wasser.

Eine Desinfektionsbehandlung kann insbesondere durch eine thermische Desinfektion stattfinden, indem das eingesetzte Reinigungs- und/oder Desinfektionsmittel auf eine vorbestimmte Mindesttemperatur aufgeheizt wird. Mit ein und demselben Reinigungs- und/oder Desinfektionsmittel kann so eine Reinigung als auch eine Desinfektion durchgeführt werden.

Obgleich sich vorbekannte Reinigungs- und/oder Desinfektionsgeräte im alltäglichen Praxiseinsatz bewährt haben, besteht Verbesserungsbedarf. Es ist insbesondere eine noch weiter vereinfachte Handhabung angestrebt, und dies bei gleichzeitiger Erhöhung der Prozesssicherheit. Es ist deshalb ausgehend vom vorbeschriebenen Stand der Technik die **Aufgabe** der Erfindung, ein Reinigungs- und/oder Desinfektionsgerät der gattungsgemäßen Art konstruktiv dahingehend weiterzuentwickeln, dass bei gleichzeitiger Verbesserung der Prozesssicherheit eine vereinfachte Handhabung durch einen Verwender ermöglicht ist.

Zur **Lösung** dieser Aufgabe wird mit der Erfindung ein Reinigungs- und/oder Desinfektionsgerät der eingangs genannten Art vorgeschlagen, das sich auszeichnet durch eine mit der Beschickungseinrichtung in strömungstechnischer Wirkverbindung stehende Medienschnittstelle, die medieneingangsseitig an eine Zuführleitung für das Reinigungs- und/oder Desinfektionsmittel, an eine Zuführleitung für Druckluft und an eine Zuführleitung für ein Pflegemittel strömungstechnisch angeschlossen ist.

Die Beschickungseinrichtung des erfindungsgemäßen Reinigungs- und/oder Desinfektionsgerätes ist an eine Medienschnittstelle strömungstechnisch angeschlossen. Die Beschickungseinrichtung steht insofern in strömungstechnischer Verbindung mit einer Medienschnittstelle.

Die Medienschnittstelle dient dazu, die Beschickungseinrichtung mit unterschiedlichen Medien zu beaufschlagen, und zwar vorzugsweise programmgesteuert, d.h. in Abhängigkeit des vom Reinigungs- und/oder Desinfektionsgeräts im bestimmungsgemäßen Verwendungsfall durchgeführten Reinigungs- und/oder Desinfektionsprogramms.

Die Medienschnittstelle ich ihrerseits erfindungsgemäß medieneingangsseitig an eine Zuführleitung für das Reinigungs- und/oder Desinfektionsmittel, an eine Zuführleitung für Druckluft und an eine Zuführleitung für ein Pflegemittel strömungstechnisch angeschlossen. Es kann mithin unter strömungstechnischer Zwischenordnung der Medienschnittstelle eine wahlweise Beaufschlagung der Beschickungseinrichtung mit einem Reinigungs- und/oder Desinfektionsmittel, Druckluft und/oder einem Pflegemittel stattfinden. Konstruktiv ist es so in vorteilhafter Weise ermöglicht, in nur einem Aufbereitungsvorgang ein vom Reinigungs- und/oder Desinfektionsgerät aufgenommenes Hohlkörperinstrument von außen und innen zu reinigen und zu desinfizieren, zu trocknen bzw. den Reinigungs- und/oder Desinfektionsschritt mittels Druckluft zu unterstützen, sowie durch Einbringen eines entsprechenden Pflegemittels für eine bestimmungsgerechte und langlebige Verwendung pflegen zu können. Dabei besteht ferner die Möglichkeit, das Einbringen des Pflegemittels unter Drucklufteinwirkung durchzuführen, so dass bei einer gleichzeitigen Minimierung der eingesetzten Menge an Pflegemittel eine verwendungsgerechte Pflege sichergestellt ist.

Als Pflegemittel kommt insbesondere ein Pflegeöl zum Einsatz, vorzugsweise ein medizinisches Wißöl. Dieses wird nach einer abgeschlossenen Reinigung und/oder Desinfektion des Hohlkörperinstruments in den vom Hohlkörperinstrument bereitgestellten Hohlraum eingebracht. Dies ist für eine bestimmungsgemäß weitere Verwendung des Hohlkörperinstruments zwingend erforderlich, insbesondere bei dentalen Übertragungsinstrumenten, wie z.B. Winkelstücken, Handstücken oder Turbinen, weil es ansonsten zu einem Anbacken oder Festfressen der vom Hohlraum aufgenommen Drehteile kommen kann. Insoweit ist eine vorbeschriebene Pflege durch Einbringen eines entsprechenden Pflegemittels in den von einem Hohlkörperinstrument bereitgestellten Hohlraum unabdingbar. Nach dem Stand der Technik geschieht dies entweder manuell oder in einem separaten Behandlungsschritt mittels eines dafür vorgesehenen Behälters oder Autoklaven, wie dieser beispielsweise aus der EP 2 614 840 B1 bekannt ist.

Sowohl eine manuell als auch eine mittels eines separaten Behälters oder Autoklaven durchzuführende Pflege bedeutet einen verwenderseitig erhöhten Aufwand als auch eine Minimierung der Prozesssicherheit. So ist die Qualität einer manuellen Pflege nicht reproduzierbar und im Übrigen vom Geschick und der Gründlichkeit der die Pflege durchführenden Person abhängig. Eine automatisierte Pflege mittels eines hierfür vorgesehenen Behälters oder Autoklaven mag diesbezüglich eine gewisse Abhilfe schaffen, doch die Praxis hat gezeigt, dass ein solch zusätzlich durchzuführender Pflegeschritt nicht immer durchgeführt wird, sei es, dass er vom Bedienpersonal als unnötig angesehen oder gar vergessen wird. In jedem Fall aber haftet der vorbekannten Prozessführung der Nachteil an, dass im Anschluss einer bestimmungsgemäßen Instrumentenreinigung eine Pflege derselben in einem separaten Verfahrensschritt durchzuführen ist. Dies ist vergleichsweise umständlich und zeitaufwendig.

Die erfindungsgemäße Hilfe schafft hier Abhilfe und überwindet die voraufgeführten Nachteile allesamt. So ermöglicht ein erfindungsgemäßes Reinigungs- und/oder Desinfektionsgerät in nur einer Verfahrensdurchführung eine vollständige Aufbereitung, und zwar einschließlich einer bestimmungsgemäßen Pflege. Das nach Abschluss eines Aufbereitungsverfahrens dem Reinigungs- und/oder Desinfektionsgerät zu entnehmende Hohlkörperinstrument ist in vorteilhafter Weise verwendungsfertig, d.h. nicht nur gereinigt und/oder desinfiziert, sondern auch gepflegt. Es ist so eine insgesamt vereinfachte Handhabung gewährleistet, da es keines zusätzlichen Pflegeschritts mehr bedarf. Darüber hinaus ist die Prozesssicherheit wesentlich verbessert, da das Pflegeergebnis reproduzierbar ist und es im Übrigen auch sicher ausgeschlossen ist, dass der Pflegeschritt infolge einer verwenderseitigen Fehlbedienung unterbleibt.

Mit Bezug auf die erfindungsgemäße Ausgestaltung stellt sich zudem ein synergetischer Effekt insoweit ein, als dass eine Druckluftbeaufschlagung des Hohlkörperinstruments nicht nur für eine verbesserte Reinigung oder Trocknung genutzt werden kann, sondern auch dazu, die eingesetzte Menge an Pflegemittel zu minimieren. Denn durch die zusätzliche Möglichkeit, auch während eines Pflegeschritts Druckluft einsetzen zu können, bedarf es zur vollständigen Pflege des gesamten von einem Hohlkörperinstrument bereitgestellten Hohlraums nicht einer Flutung desselben mit einem Reinigungsmittel. Vielmehr ist nur der Einsatz einer verringerten Pflegemittelmenge möglich, das dann druckluftbeaufschlagt innerhalb des gesamten Hohlraums gleich verteilt werden kann. Im Ergebnis wird so bei einer gleichzeitigen Verringerung der eingesetzten Pflegemittelmenge eine verwendungsgerechte Gleichverteilung des eingesetzten Pflegemittels erreicht. Dies gilt nicht nur im Besonderen für dentaltechnische Übertragungsinstrumente, die mechanisch arbeiten, wie zum Beispiel Hand- oder Winkelstücke, sondern auch für solche, die pneumatisch bedient werden, wie beispielsweise Turbinen.

Im Ergebnis erbringt die erfindungsgemäße Ausgestaltung insbesondere drei wesentliche Vorteile. Es ist kein separates, zusätzliches Pflegegerät erforderlich. Sowohl die Reinigung und Desinfektion als auch die Pflege finden in ein und demselben Reinigungs- und/oder Desinfektionsgerät statt. Es wird ferner eine verbesserte Prozesssicherheit gewährleistet, weil einerseits eine reproduzierbare Pflege gewährleistete ist und weil andererseits der Pflegeschritt der Ölpflege seitens des Bedienpersonals nicht mehr vergessen werden kann. Insofern trägt die erfindungsgemäße Ausgestaltung auch dazu bei, dass zusätzliche Kosten durch eine Ersatzbeschaffung von infolge mangelnder Pflege nicht mehr bestimmungsgemäß verwendbarer Instrumente vermieden sind. Des Weiteren ist eine Verbesserung der Benutzerfreundlichkeit erreicht, insbesondere aufgrund einer erzielten Zeitersparnis durch Verkürzung der Prozesskette und durch Vermeidung eines manuellen Handlings.

Gemäß einem weiteren Merkmal der Erfindung ist vorgesehen, dass die Beschickungseinrichtung einen mit der Medienschnittstelle in strömungstechnischer Wirkverbindung stehenden Ausgangsanschluss für den strömungstechnischen Anschluss des Hohlkörperinstruments aufweist, wobei der Ausgangsanschluss drei Ausgangskanäle bereitstellt, wobei einer der Kanäle mit dem Reinigungs- und/oder Desinfektionsmittel, der Druckluft und/oder dem Pflegemittel beaufschlagbar ist und die beiden anderen Kanäle jeweils mit dem Reinigungs- und/oder Desinfektionsmittel und/oder der Druckluft beaufschlagbar sind.

Die Beschickungseirichtung verfügt über einen Ausgangsanschluss. Dieser Ausgangsanschluss dient dazu, ein aufzubereitendes Hohlkörperinstrument an die Beschickungseinrichtung strömungstechnisch anschließen zu können. Dabei ist ein Hohlkörperinstrument im bestimmungsgemäßen Verwendungsfalls auswechselbar an einem Ausgangsanschluss angeordnet. Nach einer abgeschlossenen Aufbereitung kann das Hohlkörperinstrument mithin für eine bestimmungsgemäße Verwendung vom Ausgangsanschluss getrennt werden. Alsdann kann eine Neubestückung des Ausgangsanschlusses mit einem noch aufzubereitenden Hohlkörperinstrument stattfinden.

Der Ausgangsanschluss stellt drei Ausgangskanäle bereit. Diese Ausgangskanäle werden im bestimmungsgemäßen Verwendungsfall durch die Medienschnittstelle mit einem Medium beaufschlagt. Dabei ist vorgesehen, dass einer der drei Ausgangskanäle mit einem Reinigungs- und/oder Desinfektionsmittel, Druckluft und/oder einem Pflegemittel beaufschlagt werden kann, insbesondere wahlweise. Die beiden anderen Kanäle sind indes jeweils nur mit dem Reinigungs- und/oder Desinfektionsmittel und/oder der Druckluft beaufschlagbar, d.h. nicht mit dem Pflegemittel.

Es ist so eine wahlweise, d.h. im bestimmungsgemäße Verwendungsfall programmgesteuerte Beaufschlagung der einzelnen Kanäle mit unterschiedlichen Medien gestattet. Dabei findet im bestimmungsgemäßen Verwendungsfall typischerweise zunächst eine Reinigung und Desinfektion des Hohlkörperinstruments statt, bevor dann in einem abschließenden Pflegeschritt eine Pflege des Hohlkörperinstruments durch eine Pflegemittelbeaufschlagung stattfindet.

Gemäß einem weiteren Merkmal der Erfindung ist vorgesehen, dass die Beschickungseinrichtung eine Mehrzahl von Ausgangsanschlüssen aufweist, die jeweils in strömungstechnischer Wirkverbindung mit der Medienschnittstelle stehen und die jeweils dazu eingerichtet sind, ein Hohlkörperinstrument auswechselbar aufzunehmen.

Gemäß dieser bevorzugten Ausführungsform der Erfindung kann die Beschickungseinrichtung mit einer Mehrzahl von Hohlkörperinstrumenten bestückt werden, die im bestimmungsgemäßen Verwendungsfall gleichzeitig aufbereitet werden. So kann die Beschickungseinrichtung beispielsweise über sechs, acht, zehn oder noch mehr Ausgangsanschlüsse verfügen, womit eine entsprechende Anzahl von Hohlkörperinstrumenten gleichzeitig in bestimmungsgemäßer Weise aufbereitet werden kann. Dabei dient ein Ausgangsanschluss jeweils der Anordnung eines Hohlkörperinstruments.

Gemäß einem weiteren Merkmal der Erfindung ist vorgesehen, dass die Ausgangsanschlüsse von einer Verteilkammer bereitgestellt sind. Gemäß dieser Ausgestaltung findet im bestimmungsgemäßen Verwendungsfall eine Medienzuführung in die Verteilkammer statt. Unter strömungstechnischer Zwischenordnung der Verteilkammer findet so eine Gleichbeaufschlagung sämtlicher von der Verteilkammer bereitgestellten Ausgangsanschlüsse statt. Es können zu diesem Zweck entsprechende Koppelstellen vorgesehen sein, die eine Beschickung der Verteilkammer mit nur einem Medium oder mit einer Mischung aus zwei Medien ermöglichen. In jedem Fall findet mittels der Verteilkammer eine Mediengleichbeaufschlagung der Ausgangsanschlüsse statt.

Gemäß einem weiteren Merkmal der Erfindung ist in diesem Zusammenhang vorgesehen, dass die Verteilkammer medieneingangsseitig an zwei Koppelstellen angeschlossen ist, wobei eine erste Koppelstelle mit dem Reinigungs- und/oder Desinfektionsmittel, der Druckluft und/oder dem Pflegemittel und die zweite Koppelstelle mit dem Reinigungs- und/oder Desinfektionsmittel und/oder der Druckluft versorgt ist. Es ist so insbesondere eine Beaufschlagung der Verteilkammer mit einem Reinigungs- und/oder Desinfektionsmittel, mit Druckluft, mit einem Pflegemittel, mit einer Mischung aus einem Reinigungs- und/oder Desinfektionsmittel und Druckluft und/oder einer Mischung aus einem Pflegemittel und Druckluft ermöglicht. Über die Verteilkammer findet alsdann eine entsprechende strömungstechnische Gleichbeaufschlagung der davon bereitgestellten Ausgangsanschlüsse statt.

Gemäß einer alternativen Ausgestaltung der Erfindung ist vorgesehen, dass die Verteilkammer eine Verschlauchung beherbergt, mittels welcher die einzelnen Ausgangskanäle eines jeden Ausgangsanschlusses an einen zugehörigen Medienausgang der Medienschnittstelle strömungstechnisch angeschlossen sind. Gemäß dieser Ausgestaltung ist es im Besonderen ermöglicht, die Ausgangskanäle unterschiedlicher Ausgangsanschlüsse mit unterschiedlichen Medien oder Medienmischungen beaufschlagen zu können. Es ist so eine gleichzeitige Aufbereitung mehrerer Hohlkörperinstrumente ermöglicht, wobei die einzelnen Hohlkörperinstrumente je nach Medienbeaufschlagung unterschiedlich aufbereitet werden können. Natürlich ist auch eine Gleichbehandlung mehrerer Hohlkörperinstrumente gestattete, indem eine Mediumgleichbeaufschlagung der einzelnen Ausgangskanäle der jeweils zugehörigen Ausgangsanschlüsse stattfindet.

Gemäß einem weiteren Merkmal der Erfindung ist ein erstes schaltbares Ventil vorgesehen, das eingangsseitig an eine Zuführleitung für Druckluft und an eine Zuführleitung für ein Reinigungs- und/oder Desinfektionsmittel angeschlossen ist. Je nach Stellung dieses ersten Schaltventils findet mithin wahlweise eine Beschickung entweder mit Druckluft oder mit einem Reinigungs- und/oder Desinfektionsmittel statt. Dabei kann das Schaltventil auch pulsartig geschaltet werden, so dass sich Druckluftstöße gefolgt von Reinigungs- und/oder Desinfektionsmittelstöße ergeben.

Gemäß einem weiteren Merkmal der Erfindung ist in diesem Zusammenhang ein zweites schaltbares Ventil vorgesehen, das eingangsseitig an eine Zuführleitung für das Pflegemittel und an das erste schaltbare Ventil angeschlossen ist. Das zweite schaltbare Ventil ist mithin dem ersten schaltbaren Ventil in Strömungsrichtung nachgeschaltet. Das zweite schaltbare Ventil wird mithin je nach Stellung des ersten schaltbaren Ventils entweder mit Druckluft oder mit einem Reinigungs- und/oder Desinfektionsmittel beaufschlagt. Das zweite Schaltventil ist ferner an eine Zuführleitung für das Pflegemittel angeschlossen, so dass über das zweite Schaltventil geschaltet werden kann, ob eine Beschickung mit Pflegemittel einerseits oder mit Druckluft bzw. Reinigungs- und/oder Desinfektionsmittel andererseits erfolgt.

Über die strömungstechnische Hintereinanderschaltung von erstem Schaltventil einerseits und zweitem Schaltventil andererseits ist es mithin gestattet, eine Beaufschlagung der Medienschnittstelle mit unterschiedlichen Medien vornehmen zu können, und dies auch in einem zeitlichen Wechsel.

Gemäß einem weiteren Merkmal der Erfindung ist ein Adapterelement vorgesehen, das dazu eingerichtet ist, als Zwischenglied zwischen Ausgangsanschluss und Hohlkörperinstrument zu dienen. Das Adapterelement ist mithin im bestimmungsgemäßen Verwendungsfall strömungstechnisch zwischen dem Ausgangsanschluss und dem Hohlkörperinstrument zwischengeordnet.

Das Adapterelement stellt vorzugsweise einen Verteilraum bereit. Dieser wird medieneingangsseitig durch die ausgangsanschlussseitigen Anschlusskanäle gespeist. Medienausgangsseitig ist der vom Adapterelement bereitgestellte Verteilraum an einen adapterelementseitigen Kanal angeschlossen, so dass ein mit dem Adapterelement in Wirkverbindung stehendes Hohlkörperinstrument entweder mit einem Reinigungs- und/oder Desinfektionsmittel, Druckluft, einem Pflegemittel oder einer Mischung hiervon beaufschlagt werden kann, auch in zeitlicher Abfolge abwechselnd. Insbesondere kann eine Gleichbeaufschlagung des Verteilraums mit Pflegemittel einerseits und Druckluft andererseits stattfinden, so dass der von dem daran angeschlossenen Hohlkörperinstrument bereitgestellte Hohlraum mit einem in einem Druckluftstrom gleich verteilten Pflegemittel beaufschlagt wird.

Mit der erfindungsgemäßen Ausgestaltung wird ferner vorgeschlagen ein Verfahren zur Aufbereitung eines medizinischen, insbesondere zahnmedizinischen Hohlkörperinstruments, bei dem in einem ersten Verfahrensschritt eine Reinigung durch eine gleichzeitige Außen- und Innenbeaufschlagung des Hohlkörperinstruments mit einem Reinigungs- und/oder Desinfektionsmittel stattfindet, wobei in einem nachfolgenden Verfahrensschritt eine Innenbeaufschlagung des vom Hohlkörperinstrument bereitgestellten Hohlraums mit einem Pflegemittel und Druckluft durchgeführt wird.

Das erfindungsgemäße Reinigungs- und/oder Desinfektionsgerät ermöglicht eine erfindungsgemäße Verfahrensdurchführung, wonach vorgesehen ist, dass sowohl eine Außen- als auch eine Innenreinigung des Hohlkörperinstruments stattfindet, gefolgt von einem Pflegeschritt, demgemäß der vom Hohlkörperinstrument bereitgestellte Hohlraum mit Pflegemittel, vorzugsweise mit einer Mischung aus Pflegemittel und Druckluft beaufschlagt wird. Gemäß dieser erfindungsgemäßen Verfahrensdurchführung wird nicht nur eine Reinigung und Desinfektion des Hohlkörperinstruments ermöglicht, sondern auch eine Pflege desselben. Im Ergebnis der erfindungsgemäßen Verfahrensdurchführung steht ein endfertig aufbereitetes und damit wieder verwendbares Hohlkörperinstrument. In Abkehr zum Stand der Technik ist es nicht erforderlich, eine separate Nachbehandlung zwecks Pflege des Hohlkörperinstruments durchführen zu müssen. In schon vorerläuterter Weise wird hierdurch eine vereinfachte Handhabung für den Verwender erreicht, und dies unter gleichzeitiger Verbesserung der Prozesssicherheit. Insbesondere kann es seitens eines Bedienpersonals bei Durchführung des erfindungsgemäßen Verfahrens nicht mehr vergessen werden, nach erfolgter Reinigung und/oder Desinfektion des Hohlkörperinstruments auch eine Pflege desselben durchzuführen.

Gemäß einem weiteren Merkmal der Erfindung ist vorgesehen, dass dem Pflegeschritt ein Verfahrensschritt vorangeschaltet ist, bei dem eine Innen- und/oder Außenbeaufschlagung des Hohlkörperinstruments mit Druckluft stattfindet. Gemäß dieser bevorzugten Ausführungsform findet nach einer Reinigung und/oder Desinfektion des Hohlkörperinstruments eine Druckbeaufschlagung desselben zwecks Trocknung statt. Insbesondere etwaige Reinigungs- und/oder Desinfektionsmittelreste können so aus dem vom Hohlkörperinstrument bereitgestellten Hohlraum ausgetrieben werden. Dies ermöglicht eine verkürzte Prozessabwicklung, d.h. Verfahrensdurchführung, sowie eine sichere und gleich verteilte Pflegemittelbeaufschlagung des vom Hohlkörperinstrument bereitgestellten Hohlraums.

Gemäß einem weiteren Merkmal der Erfindung ist vorgesehen, dass der Verfahrensschritt der Reinigung in ein Vorspülen, ein Reinigen, ein Zwischenspülen und ein Nachspülen unterteilt wird. Demnach kann der Verfahrensschritt der Reinigung eine Mehrzahl von Einzelschritten umfassen, vorzugsweise je nach aufzubereitendem Hohlkörperinstrument. Dabei können je Einzelschritt sowohl unterschiedliche Medien zum Einsatz kommen, und dies auch mit unterschiedlichen Temperaturen und/oder unterschiedlichen Haltezeiten. Es kann so sowohl eine Reinigung als auch eine thermische Desinfektion durchgeführt werden, und zwar mit ein und demselben Reinigungs- und/oder Desinfektionsmittel. Insbesondere kann in diesem Zusammenhang Wasser zum Einsatz kommen, das vorzugweise mit einer Prozesschemikalie angereichert ist.

Weitere Merkmale und Vorteile der Erfindung ergeben sich aus der nachfolgenden Beschreibung anhand der Figuren. Dabei zeigen
- Fig. 1: in schematischer Darstellung ein erfindungsgemäßes Reinigungs- und/oder Desinfektionsgerät;
- Fig. 2: in schematischer Funktionsdarstellung ein erfindungsgemäßes Reinigungs- und/oder Desinfektionsgerät gemäß einer ersten Ausführungsform;
- Fig. 3: in schematischer Funktionsdarstellung ein erfindungsgemäßes Reinigungs- und/oder Desinfektionsgerät gemäß einer zweiten Ausführungsform;
- Fig. 4: in einer schematischen Detailansicht einen Ausgangsanschluss und
- Fig. 5: in schematischer Darstellung ein Reinigungs- und/oder Desinfektionsgerät nach dem Stand der Technik.

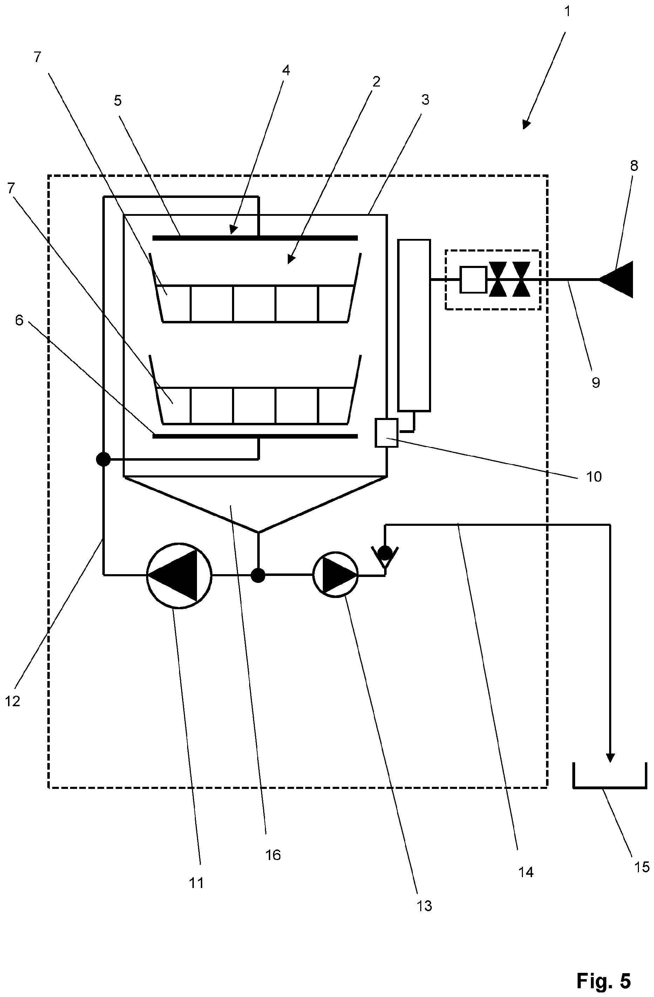

Fig. 5 lässt in einer rein schematischen Darstellung ein Reinigungs- und/oder Desinfektionsgerät 1 nach dem Stand der Technik erkennen.

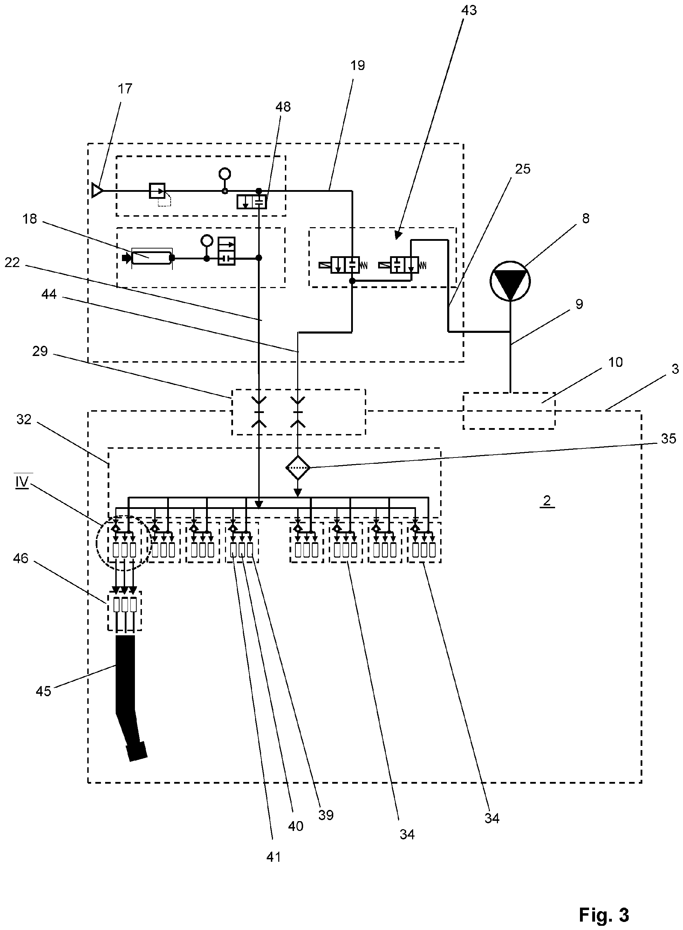

Das Reinigungs- und/oder Desinfektionsgerät 1, nachfolgend kurz Reinigungsgerät genannt, verfügt über einen Gerätebehälter 3, der einen Behandlungsraum 2 bereitstellt. Im bestimmungsgemäßen Verwendungsfall nimmt der Behandlungsraum 2 zu reinigende bzw. zu desinfizierende Spülgüter auf, so auch Hohlkörperinstrumente 45 (vgl. Fig. 3), wie z. B. dentaltechnische Übertragungsinstrumente in der Ausgestaltung als Winkelstücke, Handstücke und/oder Turbinen.

Innerhalb des Gerätebehälters 3 ist eine Sprüheinrichtung 4 angeordnet. Diese verfügt im gezeigten Ausführungsbeispiel über zwei sich im bestimmungsgemäßen Verwendungsfall drehende Sprüharme 5 und 6. Diese dienen einer Außenbeaufschlagung von zu reinigenden Spülgütern mit einem Reinigungs- und/oder Desinfektionsmittel.

Für eine Anordnung von zu reinigenden Spülgütern innerhalb des Behandlungsraums 2 kommen Spülgutträger 7 zum Einsatz, die im gezeigten Ausführungsbeispiel jeweils nach Art eines Korbs ausgebildet sind.

Als Reinigungs- und/oder Desinfektionsmittel dient im gezeigten Ausführungsbeispiel mit einer Prozesschemikalie versetztes Wasser. Das Reinigungsgerät 1 ist zu diesem Zweck unter Zwischenschaltung einer Zuführleitung 9 an einen Wasseranschluss 8 strömungstechnisch angeschlossen. Je nach Ausgestaltung des Reinigungsgerätes 1 können in die Zuführleitung 9 weitere Komponenten integriert sein. Schlussendlich mündet die Zuführleitung 9 in einen Einlauf 10, über welchen aus dem Wasseranschluss 8 stammendes Wasser in den Behandlungsraum 2 überführt wird.

An den Behandlungsraum 2 schließt sich strömungstechnisch ein Sammeltopf 16 an. Dieser steht über eine entsprechende Leitung in strömungstechnischer Verbindung mit einer Umwälzpumpe 11 einerseits und einer Abwasserpumpe 13 andererseits. Dabei schließt sich in Durchströmungsrichtung an die Umwälzpumpe 11 eine in die Sprüheinrichtung 4 einmündende Leitung 12 an. Die Abwasserpumpe 13 steht indes über eine Leitung 14 in strömungstechnischer Verbindung mit einem Abwasserkanal 15.

Im bestimmungsgemäßen Verwendungsfall findet eine Reinigung und/oder Desinfektion von im Behandlungsraum 2 befindlichen Spülgütern im Umwälzbetrieb statt. Das sich im Sammeltopf 16 ansammelnde Reinigungs- und/oder Desinfektionsmittel wird mittels der Umwälzpumpe 11 über die Leitung 12 zur Sprüheinrichtung 4 gefördert. Über die Sprüharme 5 und 6 der Sprüheinrichtung 4 findet eine Abgabe des Reinigungs- und/oder Desinfektionsmittels auf die von den Spülgutträgern 7 beherbergten Spülgüter statt, wodurch eine Außenreinigung bzw. -desinfektion derselben stattfindet. Von den Spülgütern abtropfendes Reinigungs- und/oder Desinfektionsmittel sammelt sich im Sammeltopf 16 erneut an, so dass infolge des schon vorbeschriebenen Umwälzbetriebs eine erneute Beschickung der Spülgüter mit Reinigungs- und/oder Desinfektionsmittel stattfinden kann.

Nach Abschluss einer bestimmungsgemäßen Außenreinigung und/oder -desinfektion der Spülgüter kann sich das im Sammeltopf 16 ansammelnde Reinigungs- und/oder Desinfektionsmittel durch Abpumpen mittels der Abwasserpumpe 13 verworfen werden, indem es unter strömungstechnischer Zwischenschaltung der Leitung 14 in den Abwasserkanal 15 überführt wird.

Wie sich aus der vorbeschriebenen Darstellung ergibt, findet eine Reinigung der Spülgüter dadurch statt, dass mit einer Prozesschemikalie versetztes Waser umgewälzt wird. Infolge dessen findet eine Außenbeaufschlagung der Spülgüter statt, was zu einer Reinigung derselben führt. Eine Desinfektion der Spülgüter wird durch eine thermische Desinfektion erreicht, indem das umgewälzte Wasser auf eine vorgebbare Desinfektionstemperatur aufgeheizt wird. Mit ein und derselben sogenannten Spülflotte findet mithin sowohl eine Reinigung als auch eine Desinfektion der Spülgüter statt.

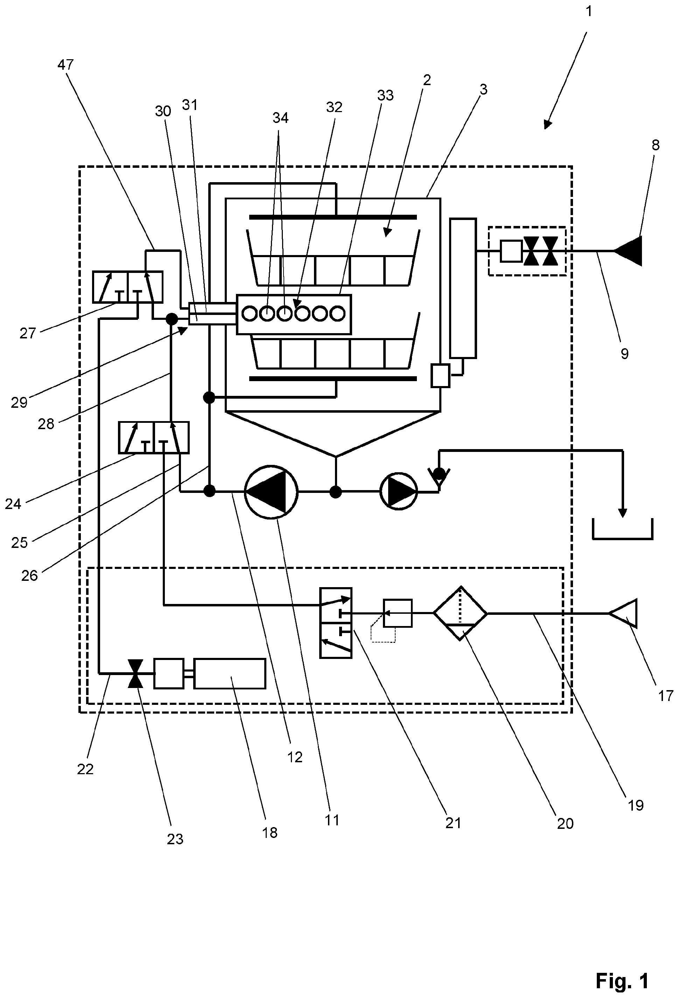

Fig. 1 lässt in schematischer Darstellung ein erfindungsgemäßes Reinigungsgerät 2 erkennen, wobei die in Fig. 1 im Vergleich zu Fig. 5 identisch verwendeten Bezugszeichen identische Bauteile oder Komponenten des Reinigungsgeräts 1 identifizieren.

Das erfindungsgemäße Reinigungsgerät 1 verfügt über eine innerhalb des Gerätebehälters 3 angeordnete Beschickungseinrichtung 32 zur Innenbeaufschlagung eines vom Hohlkörperinstrument bereitgestellten Hohlraums, insbesondere eines englumigen Kanals oder dgl., mit dem Reinigungs- und/oder Desinfektionsmittel. Die Beschickungseinrichtung 32 verfügt zu diesem Zweck, wie dies im Weiteren noch näher beschrieben werden wird, über Ausgangsanschlüsse 34 (dargestellt sind in Fig. 1 lediglich als Beispiel sechs solcher Ausgangsanschlüsse), an die jeweils ein innen zu beaufschlagendes Hohlkörperinstrument strömungstechnisch angeschlossen werden kann. Im Ergebnis dieser Ausgestaltung steht, dass ein zu reinigendes Hohlkörperinstrument 45 mittels des Reinigungs- und/oder Desinfektionsmittels sowohl außen als auch innen beaufschlagt wird, wobei für eine Außenbeaufschlagung die Sprüheinrichtung 4 und für eine Innenbeaufschlagung die Beschickungseinrichtung 32 dient.

Erfindungsgemäß ist eine mit der Beschickungseinrichtung 32 in strömungstechnischer Wirkverbindung stehende Medienschnittstelle 29 vorgesehen. Diese Medienschnittstelle 29 stellt eine erste Koppelstelle 30 sowie eine zweite Koppelstelle 31 bereit, die strömungstechnisch an eine Verteilkammer 33 der Beschickungseinrichtung 32 angeschlossen sind.

Eingangsseitig ist die Medienschnittstelle 29 an mehrere Zuführungsleitungen angeschlossen, und zwar an eine in die Zuführungsleitung 28 übergehende Leitung 26 für das Reinigungs- und/oder Desinfektionsmittel, an eine in die Zuführungsleitung 28 übergehende Leitung 19 für Druckluft und an eine Zuführungsleitung 47 für ein Pflegemittel. Die Medienschnittstelle 29 kann mithin mit dem Reinigungs- und/oder Desinfektionsmittel, der Druckluft und/oder dem Pflegemittel beaufschlagt werden. Da die Medienschnittstelle 29 in schon vorbeschriebener Weise strömungstechnisch an die Beschickungseinrichtung 32 angeschlossen ist, können jeweilige über die Ausgangsanschlüsse 34 mit der Beschickungseinrichtung 32 in strömungstechnischer Verbindung stehende Hohlkörperinstrumente 45 wahlweise mit einem Reinigungs- und/oder Desinfektionsmittel, Druckluft und/oder einem Pflegemittel innenbeaufschlagt werden.

Wie die Darstellung nach Fig. 1 des Weiteren erkennen lässt, ist zur Versorgung der Medienschnittstelle 29 mit Druckluft eine Druckluftquelle 17 vorgesehen. Diese ist mittels einer Leitung 19 strömungstechnisch an ein erstes schaltbares Ventil 24 angeschlossen. Dabei sind in die Leitung 19 ein Filter 20, ein Ventil 21 und ggf. auch weitere Baukomponenten integriert. Entscheidend ist indes allein, dass eine Versorgung der Medienschnittstelle 29 mit Druckluft durch die Druckluftquelle 17 stattfinden kann, und zwar unter Zwischenschaltung des ersten Schaltventils 24.

An das erste Schaltventil 24 ist eingangsseitig ferner der Wasseranschluss 8 strömungstechnisch angeschlossen. Zu diesem Zweck ist eine Leitung 25 vorgesehen, die der Umwälzpumpe 11 strömungstechnisch nachgeschaltet aus der Wasserleitung 12 abzweigt. Eingangsseitig des Schaltventils 24 sind mithin die Wasserleitung 25 sowie die Druckluftleitung 19 vorgesehen. Ausgangsseitig des Schaltventils 24 ist die Leitung 28 vorgesehen, die unter Zwischenschaltung einer Verteilstelle einerseits an die erste Koppelstelle 30 der Medienschnittstelle 29 und andererseits an ein zweites schaltbares Ventil 27 angeschlossen ist.

An das zweite Schaltventil 27 ist strömungstechnisch ferner eine Pflegemittelquelle 18 angeschlossen, und zwar über eine Zuführungsleitung 22. In die Zuführungsleitung 22 können weitere Komponenten integriert sein, so z.B. ein Sperrventil 23.

Bei der Pflegemittelquelle 18 kann es sich beispielsweise um eine Ölpatrone oder - kartusche handeln. Diese ist druckbeaufschlagt und gibt bei geöffneten Sperrventil 23 davon beherbergtes Öl in die Leitung 22 ab.

Am Schaltventil 27 stehen mithin eingangsseitig über die Leitung 22 ein Pflegemittel an sowie über die Leitung 28 je nach Schaltstellung des ersten Schaltventils 24 entweder Druckluft über die Leitung 19 oder ein Reinigungs- und/oder Desinfektionsmittel über die Leitung 25.

Ausgangsseitig des zweiten Schaltventils 27 ist die schon vorbeschriebene Leitung 47 vorgesehen, die in die zweite Koppelstelle 31 der Medienschnittstelle 29 mündet.

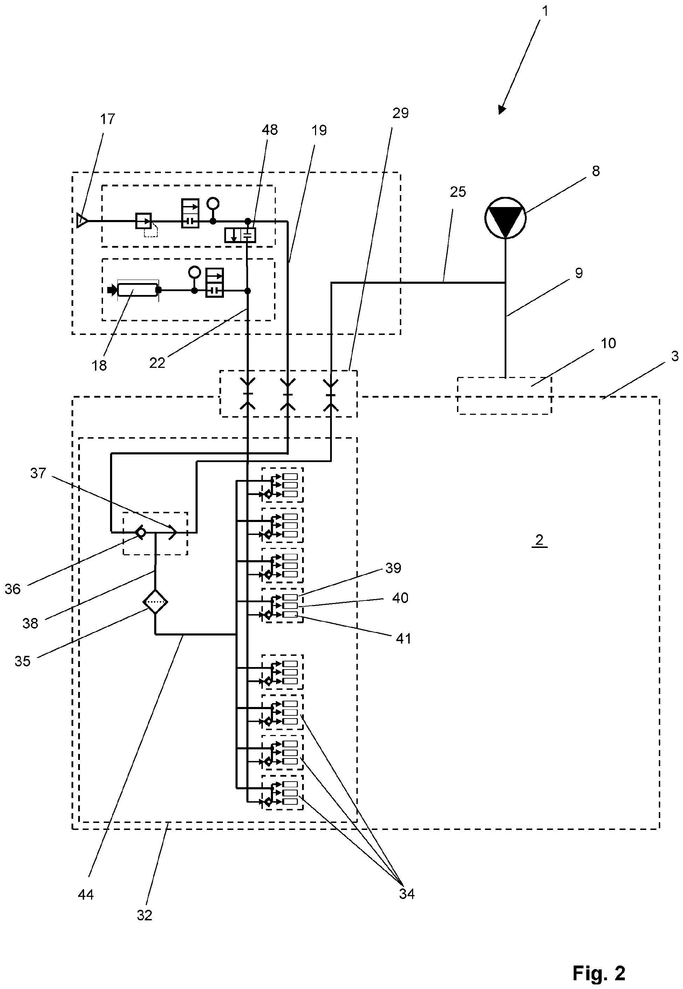

Funktionsdarstellungen weiterer erfindungsgemäßen Ausgestaltung ergeben sich aus den Figuren 2 und 3, wobei Fig. 2 eine erste Ausführungsform und Fig. 3 eine zweite Ausführungsformen zeigen.

We sich aus der Funktionsdarstellung nach Fig. 2 ergibt, stellt jeder Ausgangsanschluss 34 drei Ausgangskanäle bereit, nämlich den Ausgangskanal 39, den Ausgangskanal 40 sowie den Ausgangskanal 41. Sämtliche Ausgangskanäle 39, 40 und sind an die Leitung 44 angeschlossen, in die im gezeigten Ausführungsbeispiel ein Filter 35 integriert ist. Unter Zwischenschaltung entsprechender Ventile 36 und 37 münden in die Leitung 44 eingangsseitig die zum Wasseranschluss 8 führende Leitung 25 und die zur Druckluftquelle 17 führende Leitung 19 ein. Je nach Stellung der Ventile 36 und 37 findet mithin über die Leitung 44 eine Beaufschlagung der Ausgangskanäle 39, 40 und 41 entweder mit einem Reinigungs- und/oder Desinfektionsmittel und/oder mit Druckluft statt.

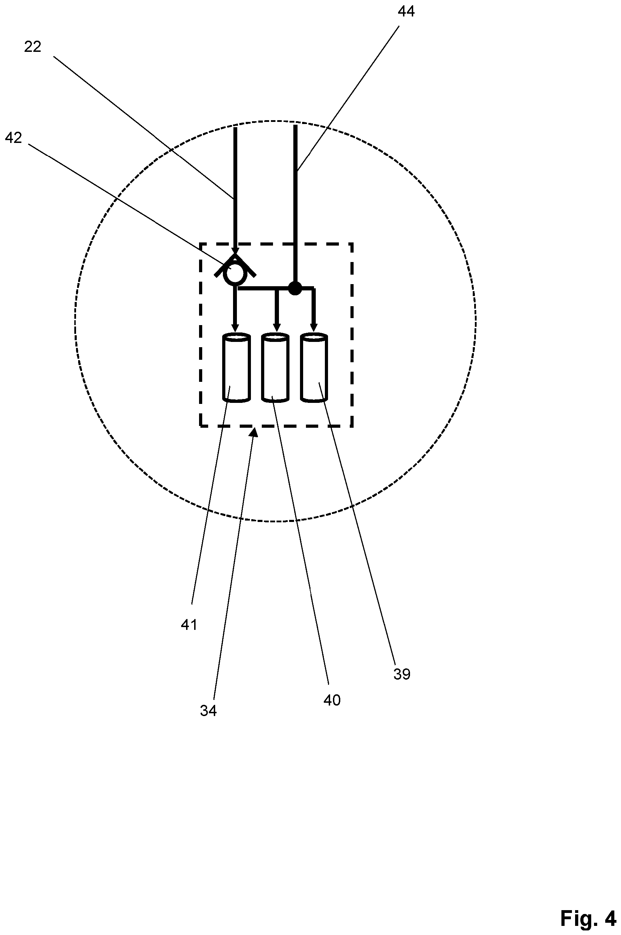

Der Ausgangskanal 41 eines jeden Ausgangsanschlusses 34 ist ferner unter Zwischenschaltung einer Leitung 22 an die Pflegemittelquelle 18 strömungstechnisch angeschlossen. Es ist mithin eine Beaufschlagung des Ausgangskanals 41 auch mit einem Pflegemittel möglich. Mittels Betätigung des an die Druckluftleitung 19 angeschlossenen Ventils 48 kann das in die Leitung 22 eingebrachte Pflegemittel druckluftunterstützt zu den jeweiligen Ausgangskanälen 41 der Ausgangsanschlüsse 34 gefördert werden. Dabei ist jedem Ausgangskanal 41 ein Rückschlagventil 42 hinsichtlich der Pflegemittelzufuhr über die Leitung 22 vorgeschaltet (vgl. Fig. 4).

Gemäß der Funktionsdarstellung nach Fig. 3 ist eine Ventilanordnung 43 vorgesehen, an die die Wasserleitung 25 einerseits und die Druckluftleitung 19 andererseits angeschlossen sind. Ausgangsseitig der Ventilanordnung 43 ist die Leitung 44 vorgesehen, die in schon vorbeschriebener Weise unter Zwischenordnung eines Filters 35 an die drei Ausgangskanäle 39, 40 und 41 eines jeden Ausgangsanschlusses 34 angeschlossen ist.

Fig. 4 lässt in einer Detailansicht die Ausgangskanäle 39, 40 und 41 eines Ausgangsanschlusses 34 erkennen, einschließlich der strömungstechnischen Anschlüsse an die Leitungen 44 und 22.

Ein jeder Ausgangsanschluss 34 dient der strömungstechnischen Anbindung eines mit einem Medium innen zu beaufschlagenden Hohlkörperinstruments 45, wie in Fig. 3 beispielhaft an einem Winkelstück dargestellt. Dabei findet ein strömungstechnischer Anschluss des Hohlkörperinstruments 45 an einen Ausgangsanschluss 34 unter Zwischenordnung eines Adapterelements 46 statt. Das Adapterelement 46 weist in Entsprechung der Ausgestaltung der drei Ausgangskanäle 39, 40 und 41 eines Ausgangsanschlusses 34 drei Eingangskanäle auf. Diese sind adapterelementseitig an einen gemeinsamen Verteilraum angeschlossen, von wo aus dann eine Mediumbeaufschlagung des vom Hohlkörperinstrument 45 bereitgestellten Hohlraums stattfindet.

Wie sich aus den Funktionsdarstellungen nach den Figuren 2 und 3 ergibt, ermöglicht das erfindungsgemäße Reinigungsgerät 1 eine gleichzeitige Mediumbeaufschlagung des zu reinigenden Hohlkörperinstruments 45 sowohl von außen als auch von innen. Dabei kann eine Innenbeaufschlagung mit einem Desinfektions- und/oder Reinigungsmittel, mit Druckluft, mit einem Pflegemittel, mit einer Mischung aus einem Reinigungs- und/oder Desinfektionsmittel mit Druckluft, einer Mischung aus einem Pflegemittel und Druckluft und/oder mit zeitlich versetzten Stößen dieser Medien stattfinden. Dies erlaubt eine verwendungsfertige Aufbereitung von Hohlkörperinstrumenten, wobei einer Reinigung und/oder Desinfektion eine Pflege durch Einbringen eines entsprechenden Pflegemittels in den Hohlraum des Hohlkörperinstruments unmittelbar nachfolgt.

Zur bestimmungsgemäßen Aufbereitung eines Hohlkörperinstruments 45 wird im Übrigen ein Verfahren vorschlagen, bei dem in einem ersten Verfahrensschritt eine Reinigung durch eine gleichzeitige Außen- und Innenbeaufschlagung des Hohlköperinstruments 45 mit einem Reinigungs- und/oder Desinfektionsmittel stattfindet. Dieser erste Verfahrensschritt kann Einzelschritte umfassen, wie z.B. Vorspülen, Reinigen, Zwischenspülen und Nachspülen. Dabei kann eine Desinfektion insbesondere während des Nachspülens stattfinden, indem das Reinigungs- und/oder Desinfektionsmittel aufgeheizt wird, beispielsweise auf eine Temperatur von über 90° C, beispielsweise 93° C. Dabei kann für eine hinreichende Desinfektion ferner eine Haltezeit vorgesehen sein, beispielsweise eine Haltezeit von mehr als 200 sec., beispielsweise 300 sec.

In einem nachfolgenden Verfahrensschritt wird dann eine Innenbeaufschlagung des vom Hohlkörperinstrument bereitgestellten Hohlraums mit einem Pflegemittel durchgeführt. Vorzugsweise findet eine Innenbeaufschlagung mit einem Druckluftstoß statt, in dem das Pflegemittel gleich verteilt ist.

Um eine wunschgemäße Gleichverteilung des Pflegemittels innerhalb des Hohlraums zusätzlich zu unterstützen, ist es bevorzugt, dem Pflegeschritt einen Trocknungsschritt vorzuschalten. Gemäß diesem Trocknungsschritt wird der mit einem Pflegemittel zu behandelnde Hohlraum zuvor mittels Druckluft beaufschlagt und ausgeblasen, so dass etwaige Wasser- bzw. Reinigungs- und/oder Desinfektionsmittelanhaftungen entfernt werden.

Die erfindungsgemäße Verfahrensdurchführung zeichnet sich gegenüber dem Stand der Technik mithin insbesondere dadurch aus, dass nach einer bestimmungsgemäßen Innenreinigung und -desinfektion eine Druckbeaufschlagung des Hohlraums stattfindet, um Flüssigkeitsreste zu entfernen. Alsdann findet unter gleichzeitiger Druckluftverwendung ein Einbringen eines Pflegemittels in den Hohlraum des Hohlkörperinstruments 45 statt. Zu diesem Zweck werden die entsprechenden Ventile für vorgebbare Öffnungszeiten von bis z.B. 5 sec geöffnet, so dass in schon vorbeschriebener Weise eine Beschickung des Hohlraums mit Pflegemittel, Druckluft und/oder einer Mischung aus Pflegemittel und Druckluft stattfinden kann. Im Ergebnis der erfindungsgemäßen Verfahrensdurchführung steht ein endfertig aufbereitetes Hohlkörperinstrument 45, das ohne weitere Nachbearbeitung einsatzbereit ist.

### Bezugszeichen

- 1: Reinigungs- und/oder Desinfektionsgerät
- 2: Behandlungsraum
- 3: Gerätebehälter
- 4: Sprüheinrichtung
- 5: Sprüharm
- 6: Sprüharm
- 7: Spülgutträger
- 8: Wasseranschluss
- 9: Zuführleitung
- 10: Einlauf
- 11: Umwälzpumpe
- 12: Leitung
- 13: Abwasserpumpe
- 14: Leitung
- 15: Abwasserkanal
- 16: Sammeltopf
- 17: Druckluftquelle
- 18: Pflegemittelquelle
- 19: Leitung
- 20: Filter
- 21: Ventil
- 22: Leitung
- 23: Sperrventil
- 24: erstes Schaltventil
- 25: Leitung
- 26: Leitung
- 27: zweites Schaltventil
- 28: Leitung
- 29: Medienschnittstelle
- 30: erste Koppelstelle
- 31: zweite Koppelstelle
- 32: Beschickungseinrichtung
- 33: Verteilkammer
- 34: Ausgangsanschluss
- 35: Filter
- 36: Ventil
- 37: Ventil
- 38: Leitung
- 39: Ausgangskanal
- 40: Ausgangskanal
- 41: Ausgangskanal
- 42: Rückschlagventil
- 43: Ventilanordnung
- 44: Leitung
- 45: Hohlkörperinstrument
- 46: Adapterelement
- 47: Leitung
- 48: Ventil

## Patentansprüche

1. Reinigungs- und/oder Desinfektionsgerät zur Aufbereitung eines medizinischen, insbesondere zahnmedizinischen Hohlkörperinstruments, mit einem einen Behandlungsraum (2) bereitstellenden Gerätebehälter (3), der der Aufnahme des aufzubereitenden Hohlkörperinstruments (45) dient, mit einer innerhalb des Gerätebehälters (3) angeordneten Sprüheinrichtung (4) zur Außenbeaufschlagung des Hohlkörperinstruments (45) mit einem Reinigungs- und/oder Desinfektionsmittel, und mit einer innerhalb des Gerätebehälters (3) angeordneten Beschickungseinrichtung (32) zur Innenbeaufschlagung eines vom Hohlkörperinstrument (45) bereitgestellten Hohlraums, insbesondere eines englumigen Kanals oder dgl., mit dem Reinigungs- und/oder Desinfektionsmittel, **gekennzeichnet durch** eine mit der Beschickungseinrichtung (32) in strömungstechnischer Wirkverbindung stehende Medienschnittstelle (29), die medieneingangsseitig an eine Zuführleitung (25, 44) für das Reinigungs- und/oder Desinfektionsmittel, an eine Zuführleitung (19, 44) für Druckluft und an eine Zuführleitung (22) für ein Pflegemittel strömungstechnisch angeschlossen ist.

2. Gerät nach Anspruch 1,
**dadurch gekennzeichnet, dass**
die Beschickungseinrichtung (32) einen mit der Medienschnittstelle (29) in strömungstechnischer Wirkverbindung stehenden Ausgangsanschluss (34) für den strömungstechnischen Anschluss des Hohlkörperinstruments (45) aufweist, wobei der Ausgangsanschluss (34) drei Ausgangskanäle (39, 40, 41) bereitstellt, wobei einer der Kanäle (41) mit dem Reinigungs- und/oder Desinfektionsmittel, der Druckluft und/oder dem Pflegemittel beaufschlagbar ist und die beiden anderen Kanäle (39, 40) jeweils mit dem Reinigungs- und/oder Desinfektionsmittel und/oder der Druckluft beaufschlagbar sind.

3. Gerät nach Anspruch 2,
**dadurch gekennzeichnet, dass**
die Beschickungseinrichtung (32) eine Mehrzahl von Ausgangsanschlüssen (34) aufweist, die jeweils in strömungstechnischer Wirkverbindung mit der Medienschnittstelle (29) stehen und die jeweils dazu eingerichtet sind, ein Hohlkörperinstrument (45) auswechselbar aufzunehmen.

4. Gerät nach Anspruch 3,
**dadurch gekennzeichnet, dass**
die Ausgangsanschlüsse (34) von einer Verteilkammer (33) bereitgestellt sind.

5. Gerät nach Anspruch 4,
**dadurch gekennzeichnet, dass**
die Verteilkammer (33) medieneingangsseitig an zwei Koppelstellen (30, 31) angeschlossen ist, wobei eine erste Koppelstelle (30) mit dem Reinigungs- und/oder Desinfektionsmittel, der Druckluft und/oder dem Pflegemittel und die zweite Koppelstelle (31) mit dem Reinigungs- und/oder Desinfektionsmittel und/oder der Druckluft versorgt ist.

6. Gerät nach Anspruch 5,
**dadurch gekennzeichnet, dass**
die Verteilkammer (33) eine Verschlauchung beherbergt, mittels welcher die einzelnen Ausgangskanäle (39, 40, 41) eines jeden Ausgangsanschlusses (34) an einen zugehörigen Medienausgang der Medienschnittstelle (29) strömungstechnisch angeschlossen sind.

7. Gerät nach einem der vorhergehenden Ansprüche,
**gekennzeichnet durch**
ein erstes schaltbares Ventil (24), das eingangsseitig an eine Zuführleitung (19) für Druckluft und an eine Zuführleitung (25) für ein Reinigungs- und/oder Desinfektionsmittel angeschlossen ist.

8. Gerät nach Anspruch 7,
**gekennzeichnet durch**
ein zweites schaltbares Ventil (27), das eingangsseitig an eine Zuführleitung (22) für das Pflegemittel und an das erste schaltbare Ventil (24) angeschlossen ist.

9. Gerät nach einem der vorhergehenden Ansprüche,
**gekennzeichnet durch**
ein Adapterelement (46) zur Zwischenordnung zwischen einem Ausgangsanschluss (34) und einem Hohlkörperinstrument (45).

10. Verfahren zur Aufbereitung eines medizinischen, insbesondere zahnmedizinischen Hohlkörperinstruments, insbesondere unter Verwendung eines Reinigungs- und/oder Desinfektionsgeräts nach einem der vorhergehenden Ansprüche, bei dem in einem Verfahrensschritt eine Reinigung durch eine gleichzeitige Außen- und Innenbeaufschlagung des Hohlkörperinstruments (45) mit einem Reinigungs- und/oder Desinfektionsmittel stattfindet, **dadurch gekennzeichnet, dass** in einem nachfolgenden Verfahrensschritt eine Innenbeaufschlagung des vom Hohlkörperinstruments (45) bereitgestellten Hohlraums mit einem Pflegemittel und Druckluft durchgeführt wird.

11. Verfahren nach Anspruch 10,
**dadurch gekennzeichnet, dass**
dem Pflegeschritt ein Verfahrensschritt vorgeschaltet ist, bei dem eine Innen- und/oder Außenbeaufschlagung des Hohlkörperinstruments (45) mit Druckluft stattfindet.

12. Verfahren nach Anspruch 10 oder 11,
**dadurch gekennzeichnet, dass**
der Verfahrensschritt der Reinigung in ein Vorspülen, ein Reinigen, ein Zwischenspülen und ein Nachspülen unterteilt wird.
